# EUROPEAN PATENT APPLICATION

(11) **EP 4 644 487 A1**
(43) Date of publication of application: **05.11.2025**
(21) Application number: 23912248.4
(22) Date of filing: 27.12.2023
(51) Int. Cl.: C08L 89/00, A23L 5/00, A23L 29/275, A61F 13/53, A61K 8/64, A61K 9/06, A61K 47/46, A61L 15/32, A61L 15/60, A61Q 19/00, D01B 7/00

(54) **GEL CONTAINING BAGWORM SILK AND METHOD FOR PRODUCING SAME**

(30) Priority: 27.12.2022 JP 2022210021
(71) Applicant: NATIONAL AGRICULTURE AND FOOD RESEARCH ORGANIZATION, Tsukuba-shi, Ibaraki 305-8517 (JP); KOWA COMPANY, LTD., Naka-ku Nagoya-shi Aichi 460-8625 (JP)
(72) Inventor: KAMBE, Yusuke, Tsukuba-shi, Ibaraki 305-8634 (JP); YOSHIOKA, Taiyo, Tsukuba-shi, Ibaraki 305-8634 (JP); KAMEDA, Tsunenori, Tsukuba-shi, Ibaraki 305-8634 (JP); ICHIDA, Yuya, Tsukuba-shi, Ibaraki 305-0856 (JP); SHIBASAKI, Manabu, Tsukuba-shi, Ibaraki 305-0856 (JP)
(74) Representative: Wächtershäuser & Hartz Patentanwaltspartnerschaft mbB
(86) International application number: PCT/JP2023/046933
(87) International publication number: WO 2024/143470

(57) **Abstract**

Provided is a new application technology of bagworm silk.

This gel includes (i) bagworm silk and (ii) water.

## Description

### Technical Field

The present invention relates to a gel containing bagworm silk and a method for producing the same.

### Background Art

Silk (bagworm silk) derived from bagworms, which is a general term for the larvae of moths belonging to the family Psychidae of the order Lepidoptera, has mechanical properties superior to those of silkworm silk and spider silk. For example, in terms of elastic modulus, the bagworm silk of tea bagworm (Eumeta minuscula) has 3.5 times as large elastic modulus as silkworm silk has, and 2.5 times as large elastic modulus as spider silk of the Joro-spider (Nephila clavata) has, and has a very high strength (Non-Patent Literatures 1 and 2).

Also in terms of rearing, bagworms have advantages over silkworms. For example, silkworms, in principle, feed only on fresh leaves of species of mulberry (the genus Morus) such as M. bombycis, M. alba, and M. Ihou, and therefore a rearing region and a rearing period thereof are dependent on a supply area of mulberry leaves and the leaf-opening period of mulberry. Meanwhile, bagworms are polyphagous, have low specificity for prey leaves, and can feed on leaves of various tree species. Therefore, the feed leaves are easily available, and the rearing regions are not restricted. In addition, depending on the species, leaves of evergreen trees can also be used as feed leaves, and therefore feed leaves can be supplied throughout the year unlike the mulberry which is a deciduous tree. Furthermore, since bagworms are smaller in size than silkworms, bagworms can be reared in a space equal to or less than that of silkworms, making mass rearing easier. Therefore, costs for rearing bagworms can be significantly reduced as compared with costs for rearing silkworms.

Also, in terms of productivity, bagworms have advantages over silkworms. For example, silkworms spin a large amount of silk only at the time of cocoon spinning, and cocoon spinning is performed in the same period for all the larvae. Therefore, there are problems that silk harvesting schedules overlap and labor periods are concentrated. Meanwhile, bagworms repeatedly spin silk during nesting or moving throughout the larval stage thereof. Therefore, there is an advantage that the labor period can be distributed by artificially adjusting the silk harvesting schedule.

As described above, bagworm silk has characteristics exceeding those of conventional animal fibers, and has many advantages in production, and thus is expected to be an environmentally friendly alternative material of silk (Non-Patent Literature 2). For example, application to a fiberreinforced composite material combined with another polymer has been reported (Patent Literature 1).

### Citation List

### Patent Literatures

Patent Literature 1: JP 2019-44117 A

### Non-Patent Literature

Non-Patent Literature 1: Nature communications (2019) 10:1469
Non-Patent Literature 2: Adhesive Technology Vol.39, N0.4 (2020): 14-17

### Summary of Invention

### Technical Problem

However, a conventional application technology of bagworm silk includes only a utilization of properties of bagworm silk as a fiber.

Therefore, an object of the present invention is to provide a new application technology of bagworm silk.

### Solution to Problem

Therefore, the present inventors examined changing bagworm silk not into the form of fibers but into other forms, to find that, quite unexpectedly, a hydrogel (bagworm silk hydrogel) was easily formed by heating and then cooling a dispersion or an aqueous solution which contains bagworm silk and adjusting a water content as necessary, and the obtained hydrogel is excellent in stability and moldability and can be used in various technical fields, and to complete the present invention.

That is, the present invention provides the following inventions [1] to [6].
[1] A gel including: (i) bagworm silk; and (ii) water (hereinafter, may be abbreviated as a bagworm silk hydrogel).
[2] The bagworm silk hydrogel according to [1], wherein a content of the water is 80% by mass or more and 99.99% by mass or less in a total amount of the gel.
[3] The bagworm silk hydrogel according to [1] or [2], further including: one or more components selected from the group consisting of gel-forming polymers, saccharides, sugar alcohols, alcohols, polyhydric alcohols, water-soluble polymers, and basic substances.
[4] The bagworm silk hydrogel according to [1], wherein the bagworm silk is silk spun by Eumeta japonica, Eumeta minuscula, or Nipponopsyche fuscescens.
[5] A food, a pharmaceutical product, a cosmetic product, a biomaterial, a cushioning material, an elastic material, a fishing tool, a medical device, an industrial material, or a sanitary material including: the bagworm silk hydrogel according to any one of [1] to [4].
[6] A method for producing the bagworm silk hydrogel according to any one of [1] to [3] including: heating a bagworm silk-containing aqueous solution to 50°C or higher, and subsequently cooling it.

### Advantageous Effects of Invention

The bagworm silk hydrogel of the present invention is a hydrogel formed from bagworm silk, which has remarkably high elasticity and strength, and water molecules. The strength or the like of the bagworm silk hydrogel can be changed. Therefore, the bagworm silk hydrogel can be used in various fields including foods, pharmaceutical products, cosmetic products, biomaterials, cushioning materials, elastic materials, fishing tools, medical devices, industrial materials, sanitary materials.

### Brief Description of Drawings

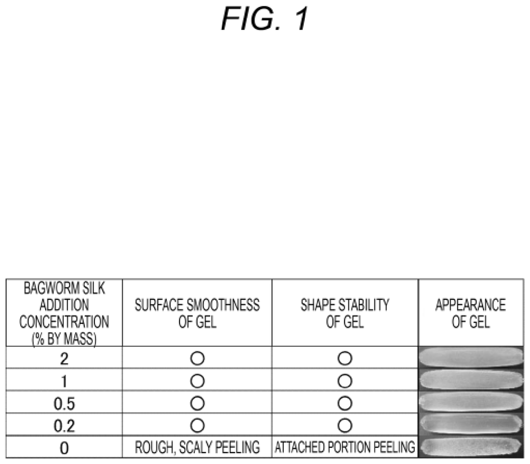

Fig. 1 shows evaluation results of surface smoothness, shape stability, and appearance of a bagworm silk hydrogel of the present invention obtained in Example 3.

### Description of Embodiments

The present invention relates to a bagworm silk hydrogel, and one aspect thereof is a bagworm silk hydrogel containing (i) bagworm silk and (ii) water.

In the present specification, a gel refers to a solid form among colloids of a liquid dispersion medium. Due to being a solid form, a gel has a property of not flowing and moving, unlike a gas or a liquid. Therefore, substances having thixotropy, which exhibit fluidity when subjected to shear stress and lose fluidity in a stationary state, are gels.

In the present specification, a hydrogel refers to a solid form of a colloid in which a dispersion medium is water. Specifically, it is in a state in which a polymer with a mesh-like structure has absorbed a large amount of water.

The bagworm silk hydrogel is a gel in a state in which bagworm silk has absorbed a large amount of water.

A main component constituting the hydrogel of the present invention is bagworm silk, thread derived from bagworms (bagworm silk). The bagworm is a generic term for the larvae of moths belonging to the family Psychidae of the order Lepidoptera. More specifically, it is a protein thread that is spun by larvae of bagworms.

The family Psychidae includes genera such as Acanthopsyche, Anatolopsyche, Bacotia, Bambalina, Canephora, Chalioides, Dahlica, Diplodoma, Eumeta, Eumasia, Kozhantshikovia, Mahasena, Nipponopsyche, Paranarychia, Proutia, Psyche, Pteroma, Siederia, Striglocyrbasia, Taleporia, Theriodopteryx, and Trigonodoma, and a bagworm referred to herein may be a species belonging to any genus. Specific examples of the species in Psychidae include Eumeta japonica, Eumeta minuscula, and Nipponopsyche fuscescens. The instar of the larvae may be any stage from the first instar to the final instar. However, for the purpose of obtaining thicker and longer bagworm silk, it is preferable to use larger bagworms. For example, in a case of the same species, the final stage larvae are more preferable, and in a case of males or females, the larger females are more preferable. In addition, in the family Psychidae, larger species are more preferable. Therefore, Eumeta japonica and Eumeta minuscula are suitable species as the bagworms.

In the present specification, the "silk" refers to a thread derived from an insect and a protein thread that is spun by insect larvae or adult insects for the purpose of, for example, nesting, moving, fixing, cocoon spinning, and bait capturing. In the present specification, when simply silk is used, it means bagworm silk unless otherwise specified.

The bagworm silk of the present specification includes single fibers, spun fibers, and aggregate fibers.

The "single fiber" is a filament of a minimum unit constituting a fiber component, and is also called a monofilament. The single fiber is mainly composed of fibroin-like proteins. The bagworm silk is naturally spun with bifilaments and usually not present as single fibers. However, by going through a degumming step, adhesive substances are removed, and a single fiber can be obtained.

The "spun fiber" is silk in a state of being spun by bagworms. The spun fiber by bagworms is composed of a bifilament consisting of a pair of two single fibers. This form is based on a form in which two single fibers discharged from spinnerets respectively located on the right and left sides of a bagworm are bonded by a sericin-like adhesive substance during spinning. In addition, in the present specification, when the term is used with "spin (spun)" as in "bagworm silk spun" and "spinning bagworm silk", this generally means a spun fiber.

The "aggregate fiber" is a fiber composed of a plurality of fiber bundles, and is also called a multifilament. This is a so-called raw silk, which is composed of a plurality of single fibers in principle, however, in the present specification, a case of being composed of a plurality of single fibers and spun fibers or composed of a plurality of spun fibers is also included. The aggregate fiber in the present specification means an aggregate fiber composed only of bagworm silk. The aggregate fiber is twisted through a twisting step to be stronger silk. However, the aggregate fiber in the present specification includes not only twisted yarn fibers but also non-twisted yarn fibers which are soft and smooth to touch.

The bagworm silk includes foothold silk thread and nest silk thread. The "foothold silk thread" is silk spun by bagworm before movement, and has a function as a scaffold for preventing the bagworm from falling from branches, leaves, and the like during movement. In general, bagworms move in a traveling direction using the foothold silk thread as a foothold and hooking their leg claws. The foothold silk thread is spun in a zigzag manner in order to make it easy for bagworms to hook their left and right legs and to distribute the load on the silk and fixing points of the silk to the left and right. Meanwhile, the "nest silk thread" is silk that forms a nest, and is spun to bind leaf pieces or twigs or to create a comfortable environment on inner walls of the nest, which serves as a residential area. In principle, the foothold silk thread is thicker and mechanically stronger than the nest silk thread.

A method for obtaining the bagworm silk is not particularly limited, and for example, it is preferable for production to use the method described in JP 2018-197415 A**.** In addition, a modified bagworm silk discharged from genetically modified silkworm described in WO 2018/074403 A can also be used.

As described in Non-Patent Literatures 1 and 2, the bagworm silk has a specific amino acid sequence and a highly ordered hierarchical structure. Such a specific amino acid sequence and a highly ordered hierarchical structure serve to formation of a fiber having both excellent elastic modulus and strength.

The bagworm silk is composed of a fibril hierarchical structure having nanofibrils as a basic unit, the nanofibrils being composed of a long-period repeating structure of a crystalline phase and an amorphous phase. The repeating unit of a primary structure is about 160 residues, which is about 5 times as large as spider silk. The greatest characteristic of the bagworm silk is that it has a hybrid form in which both a polyalanine sequence and a Gly-Gly-X (X is Ala, Tyr, or the like) sequence, which are characteristics of spider silk, and Gly-X (X is Ala, Ser, or the like), which are characteristics of silkworm, are combined. The number of amino acid residues of a crystal phase is considered to be 67 residues, and is several times as large as that of other silks (for example, silk of Muga silkworm), and the length of the period (long period) of the crystal phase and the amorphous phase, which is an indicator of the length of the crystal phase, is also several times as large as that of other silks. In the formation of a hydrogel, it is considered that such a region which is likely to form a crystal phase constitutes a physically crosslinked structure, and it is considered that constitution of a firmly crosslinked structure affects the strength of the gel.

It is considered that water molecules during formation of a hydrogel gather in an amorphous region. As described above, the repeating unit of the primary structure is 160 residues, and 67 residues among them form a crystal phase, and therefore the amorphous phase is about 90 residues. It is considered that water molecules gather in the amorphous region of 90 residues.

The bagworm silk hydrogel of the present invention contains water as a solvent or a dispersion medium. A content of water in the gel may be any amount as long as a gel can be formed at 25°C under atmospheric pressure, and can be appropriately adjusted depending on the strength, function, and the like of the intended hydrogel, as well as characteristics and the like of other additives.

The content of water in the hydrogel is preferably 80% by mass or more and 99.99% by mass or less in a total amount of the gel from the viewpoint of forming a gel at 25°C under atmospheric pressure. It is more preferably 85% by mass or more, still more preferably 90% by mass or more, and even more preferably 95% by mass or more. It is more preferably 99.95% by mass or less, still more preferably 99.9% by mass or less, and even more preferably 99.5% by mass or less.

In addition, a content mass ratio of bagworm silk (a) and water (b), (a/b), depends on the strength, function, and the like of the hydrogel, and the characteristics and the like of other additives, but is preferably 0.0001 or more and 0.1765 or less, more preferably 0.0005 or more and 0.1111 or less, and still more preferably 0.005 or more and 0.0417 or less.

In order to prepare the bagworm silk-containing aqueous solution, in addition to water, an aqueous solvent advantageous for dissolving the bagworm silk can also be used. Examples of the aqueous solvent include a solution in which a neutral salt is dissolved in water, and examples thereof include a copper-ethylenediamine aqueous solution, a copper hydroxide-ammonia aqueous solution, a copper hydroxide-alkali-glycerol aqueous solution, a lithium bromide aqueous solution, a calcium chloride aqueous solution, a magnesium chloride aqueous solution, a zinc chloride aqueous solution, a calcium nitrate aqueous solution, a magnesium nitrate aqueous solution, a zinc nitrate aqueous solution, a calcium thiocyanate aqueous solution, a magnesium thiocyanate aqueous solution, a zinc thiocyanate aqueous solution, a sodium thiocyanate aqueous solution, a lithium thiocyanate aqueous solution, a urea aqueous solution, and a sodium dodecyl sulfate aqueous solution. It is also possible to use a hydrous alcohol obtained by mixing water with a lower alcohol such as methanol or ethanol. In addition, from the viewpoint of dissolving the bagworm silk, for example, an organic solvent such as hexafluoroacetone, dichloroacetic acid, trifluoroacetic acid, hexafluoroisopropanol, hexafluoroacetone, or formic acid may be used.

The bagworm silk hydrogel of the present invention can contain various additives in consideration of the strength of the hydrogel, the function or characteristics desired to be imparted, and the like, in addition to the bagworm silk as a solute or a dispersoid, and water as a solvent or a dispersion medium.

Examples of the additives that can be contained include components having high affinity with bagworm silk and water, gel-forming polymers, saccharides, sugar alcohols, alcohols, polyhydric alcohols, water-soluble polymers, plasticizers, basic compounds such as organic bases and inorganic bases, and dyes.

Examples of the gel-forming polymers include synthetic polymers such as poly(meth)acrylic acid, poly(meth)acrylate, carboxyvinyl polymer, polyvinyl chloride, polyvinyl acetate and salts thereof, and natural polymers such as cellulose derivatives and polysaccharides (such as agar, gelatin, carrageenan, pectin, gellan gum, xanthan gum, locust bean gum, tamarind seed gum, and curdlan).

Examples of the saccharides include monosaccharides, and oligosaccharides. Examples of the sugar alcohols include erythritol, lactitol, maltitol, mannitol, sorbitol, and xylitol. Examples of the alcohols include ethanol, isopropanol, and butanol. Examples of polyhydric alcohols include ethylene glycol, polyethylene glycol, propylene glycol, polypropylene glycol, glycerol, polyglycerol, butylene glycol, and polybutylene glycol.

Examples of the water-soluble polymer include polyvinyl alcohol.

Examples of the plasticizers include triacetin, triethylene glycol diacetate, tributyl acetyl citrate, dibutyl sebacate, epoxidized soybean oil, phthalic acid ester, and adipic acid ester.

In addition, basic compounds such as organic bases or inorganic bases acts as a crosslinking agent for an acidic polymer such as polyacrylic acid.

Examples of the dyes include Food Red No. 2, Food Red No. 3, Food Red No. 40, Food Red No. 102, Food Red No. 106, Food Yellow No. 4, Food Yellow No. 5, Food Blue No. 1, Food Blue No. 2, and caramel.

Since these other components have different effects on the hydrogel of the present invention, depending on kinds thereof, a content thereof can vary greatly, but it is preferably 0.5% by mass or more and 60% by mass or less, more preferably 1% by mass or more and 45% by mass or less, still more preferably 5% by mass or more and 30% by mass or less with respect to a total amount of the gel.

The bagworm silk-containing hydrogel of the present invention can be easily produced, for example, by heating an aqueous solution or an aqueous dispersion containing bagworm silk (hereinafter, an aqueous solution or an aqueous dispersion containing bagworm silk may be abbreviated as a bagworm silk-containing aqueous solution) to 50°C or higher and then cooling the solution. In short, a concentration treatment such as dialysis, ultrafiltration, or the like may be performed on the bagworm silk-containing aqueous solution before heating.

In the method for producing the bagworm silk-containing hydrogel of the present invention, first, an aqueous solution or dispersion containing bagworm silk is prepared. The bagworm silk-containing aqueous solution can be produced by adding a sufficient amount of water, and an additive if necessary, to the bagworm silk. The additive may be added simultaneously with water, or may be added separately.

Regarding a heating temperature of the bagworm silk-containing aqueous solution, it is sufficient to be 50°C or higher, and is more preferably 60°C or higher. Also, an upper limit of the heating temperature may be 100°C or higher, and is preferably 130°C or lower in consideration of industrial productivity.

Regarding heating conditions, since it is difficult to obtain a gel having an intended property when the concentration of the bagworm silk greatly changes at the stage when the bagworm silk-containing aqueous solution is gelated, it is preferable to adopt a condition in which water is prevented from being evaporated during gelation, and a condition in which water molecules can pass through the solution or across the inside and outside of the gel. In consideration of such conditions, it is preferable to fill a container (mold) in which the water permeability is restricted to some extent with the bagworm silk-containing aqueous solution and perform heating. The container (mold) for heating is not particularly limited, and examples thereof include a dialysis membrane container, a low-density polyethylene container, and a metal container. In addition, the heating may be performed in water or in air.

After sufficient gelation is confirmed, the gel is cooled to 15°C to 25°C to obtain the bagworm silk hydrogel of the present invention.

In addition, for example, in a case where the container is a dialysis membrane, the amount of water in the gel can be adjusted by appropriately changing a solvent of an external liquid of the container.

Since no gel was obtained even when an aqueous solution of silkworm silk was heated for a long time (heated at 80°C for 500 minutes) and then cooled, a method for producing the bagworm silk-containing gel of the present invention is industrially advantageous.

In addition, to produce the bagworm silk hydrogel of the present invention, it is possible to use not only the bagworm silk but also a protein having an amino acid sequence constituting the bagworm silk or a protein having an amino acid sequence in which one or more amino acids of the amino acid sequence are deleted, added, and/or substituted based on a known method.

For example, when other components (additives) such as polyvinyl alcohol and agar are added to the bagworm silk-containing aqueous solution, and the mixture is heated to 50°C or higher and cooled, a combined hydrogel of the bagworm silk, polyvinyl alcohol, and agar is obtained. The obtained hydrogel is soft but maintains a shape, and has recoverability. In this case, when a dialysis membrane is used as a container, little water evaporates and adhesion deformation to the container does not occur.

The bagworm silk hydrogel of the present invention can also be a thixotropic gel which is solid at 15°C to 25°C but gives fluidity by applying a shear force, using other components (additives) added during adjustment of moisture and gelation. Such a gel can be used as a pharmaceutical product, a cosmetic product, a food, or the like.

It is also possible to form a hydrogel which is solid and has elasticity. Such a gel can be used, for example, in a food, a cosmetic product, a biomaterial, a cushioning material, an elastic material, a fishing tool, a medical device, an industrial material, or a sanitary material (such as a diaper).

### Examples

Next, the present invention will be described in more detail with reference to examples, but the present invention is not limited to these examples.

### Example 1

Foothold silk thread of bagworms was used as bagworm silk, and degummed in a 0.02 M aqueous sodium carbonate solution heated to 90°C for 30 minutes. 40 mg of the degummed bagworm silk was dissolved for 60 minutes in a saturated aqueous lithium thiocyanate solution heated to 70°C, then placed in a cellulose dialysis tube, and dialyzed using 2 liters of ultrapure water as a dialysate. A dialysis temperature was set to 4°C, and the dialysate was replaced four times in total at a frequency of twice a day. A concentration of the prepared bagworm silk aqueous solution was calculated to be 1.2% by mass by measuring a dry weight. The obtained bagworm silk aqueous solution was placed in a cellulose dialysis tube and air-dried and concentrated to prepare a 2% by mass bagworm silk aqueous solution. The obtained aqueous solution was enclosed in a dialysis tube and heated in a warm bath at 38°C to 80°C. The relationship between the heating time and gelation is shown in Table 1. Table 1 shows that the bagworm silk-containing aqueous solution was easily gelated when heated to 50°C or higher.

The 2% by mass silkworm silk aqueous solution did not gelate even when heated to 80°C for 500 minutes.

**[Table 1]**

| Temperature of water (°C) | Gel formation | Time when gelation started (min) | Time when gelation is complete (min) | Remarks |
|---|---|---|---|---|
| 38 | No | | | No change after 10 minutes |
| 40 | No | | | No change after 90 minutes |
| 45 | No | | | No change after 90 minutes |
| 50 | Yes | | | Gelation in 15 to 60 minutes |
| 60 | Yes | 6.5 | 8 | - |
| 70 | Yes | 3.5 | 5.5 | - |
| 80 | Yes | 1.5 | 2.5 | - |

### Example 2

A 2% by mass bagworm silk aqueous solution was enclosed in a dialysis membrane, a low-density polyethylene (LDPE) membrane, or a sealing container (a sample tube with a lid), or placed in an open container (petri dish) and heated for 20 minutes under the conditions of Table 2, and a state of the resulting gel was observed. The water bath heating and the dry bath heating were performed at 80°C. A dry heat treatment was performed by heating for 20 minutes in a dry heat sterilizer set at 80°C, and an autoclave treatment was performed by heating at 121°C for 20 minutes. The results are shown in Table 2.

From Table 2, it can be seen that a heating condition in which water molecules can pass through the inside and outside of a membrane (container) in a state where water is prevented from being evaporated during gelation, which is a favorable condition.

**[Table 2]**

| | | Container | Water evaporation | Gel cohesion | Gel recovery |
|---|---|---|---|---|---|
| Heating in water | | Enclosing in dialysis membrane | No | A: Excellent | A: Excellent |
| | | Enclosing in LDPE membrane | No | B: Good | B: Good |
| Heating in air | Dry Bath | Airtight container | Yes | B: Good | B: Good |
| | Dry heat | Open container | Yes - Large amount | C: Fair | C: Fair |
| | Autoclave | Enclosing in dialysis membrane | Yes - Small amount | A: Excellent | BB: Great |
| | | Open container | Yes - Large amount | D: Poor (Fragile) | D: Poor |

### Example 3

In the same method as in Example 1, 1 mL of bagworm silk aqueous solution prepared to have a concentration of 0.005, 0.01, 0.05, 0.07, 0.1, 0.5, 0.75, 1, 2, 4, 5, 6, or 7% by mass was enclosed in an 8/32 dialysis membrane together with air bubbles, and heated in hot water at 90°C for 15 minutes. After heating, gelation determination was performed based on the movement of the air bubbles when the dialysis membrane container was overturned, and then the dialysis membrane container was cut open for evaluating a form of the gel.

The degree of gelation was determined by the movement of air bubbles enclosed in the dialysis membrane container. The evaluation criteria are as follows.
D: Change from pre-heating cannot be confirmed.
C: Movement is slightly slower than pre-gelation.
B: Movement is slower than pre-gelation.
A: Movement of air bubble stops in the gel or air bubbles do not move.

The results are shown in Table 3.

**[Table 3]**

| Bagworm silk concentration (% by mass) | Gelation | Form |
|---|---|---|
| 0.005 | D | Liquid |
| 0.01 | C | Liquid |
| 0.05 | B | Liquid |
| 0.07 | A | Liquid |
| 0.1 | A | Liquid |
| 0.5 | A | Semi-solid |
| 0.75 | A | Solid |
| 1 | A | Solid |
| 2 | A | Solid |
| 4 | A | Solid |
| 5 | A | Solid |
| 6 | A | Solid |
| 7 | A | Solid |

| | | |
|---|---|---|
| * Degrees of gelation: A > B > C > D | | |

### Example 4

1 mL of a 10% by mass polyvinyl alcohol aqueous solution and 100 mg of agar were placed in a 5 mL sample tube, and dispersed by strong stirring with a vortex mixer. 1 mL of a 2% by mass bagworm silk aqueous solution was added, and the mixture was uniformly mixed by pipetting. The mixed solution was enclosed in a dialysis membrane container, immersed in a water bath at 90°C and heated for 30 minutes, and then further transferred to a saturated borax aqueous solution heated at 90°C and heated for 30 minutes. After removing the moisture and cooling at 4°C for 5 minutes, the dialysis membrane container was cut open to recover the gel.

The surface smoothness and shape stability of the obtained gel and the appearance of the gel were evaluated. The surface smoothness was evaluated based on the roughness of the gel surface and whether peeling occurred. The shape stability was evaluated based on whether the shape of the dialysis membrane container was maintained, peeling of a portion attached to the membrane, and the like. The appearance was evaluated based on transparency, gloss, and the like.

The results are shown in Fig. 1.

As shown in Fig. 1, a composite hydrogel of the bagworm silk of the present invention, polyvinyl alcohol, and agar was obtained, and the surface smoothness, shape stability, and appearance thereof were all favorable.

In addition, polyvinyl alcohol was not eluted by heating in water in a dialysis membrane container. Since the gel was contacted with borax water through the dialysis membrane, excess water escaped from the gel and the membrane shrunk and tightened. A gel structure can be constituted through three stages of bagworm silk gelation - > polyvinyl alcohol gelation -> agar gelation.

### Test Example 1: Comparison of physical properties between bagworm silk gel and silkworm silk hydrogel

A test was performed in order to compare the physical properties between the hydrogel containing silk of silkworm which is a spinning insect like the bagworm and the bagworm silk hydrogel of the present invention.

In the same method as in Example 1, a 2% by mass bagworm silk aqueous solution and a 2% by mass silkworm silk aqueous solution were prepared, and 2 mL of each solution was enclosed in an 8/32 dialysis membrane.

The obtained dialysis membrane container was put into a 50 mL tube containing 40 mL of ultrapure water heated in advance at 100°C setting of a dry bath, and heated to be gelated. The treatment was continued until gelation occurred by heating in water, the gel was cut to have the length of 10 mm, and a uniaxial compression test was performed (load cell: 50N, plunger: φ23mm, with the base set as Z = 0, the position where F > 0.03 N was considered as the height of the gel, measuring at a compression speed of 1mm/min up to 70% compression, and n = 9).

A compressive elasticity modulus was calculated from the stress and strain at a strain of 0.05% to 0.25%, and the results up to the time when the gel was first broken are shown in Table 4.

**[Table 4]**

| Sample name | Compressive elastic modulus (kPa) | Toughness (MJ/m3) | Fracture displacement (%) | Compressive fracture stress (kPa) |
|---|---|---|---|---|
| Bagworm silk hydrogel | 49.5 ± 14.3 | 0.92 ± 0.47 | 51.0 ± 10.5 | 43.9 ± 20.9 |
| Silkworm silk hydrogel | 61.4 ± 24.4 | 0.15 ± 0.09 | 22.5 ± 11.7 | 11.8 ± 4.9 |
| Presence or absence of significant difference (t-test) | n.s. | p < 0.001 | p < 0.001 | p < 0.001 |

The bagworm silk hydrogel had a compressive elasticity modulus of 0.8 time, a toughness of 6.0 times, a breaking displacement of 2.3 times, and a compressive breaking stress of 3.7 times, as much as the silkworm silk hydrogel, which revealed that the bagworm silk hydrogel had remarkably excellent physical properties.

## Claims

1. A gel comprising: (i) bagworm silk; and (ii) water.

2. The gel according to claim 1, wherein a content of the water is 80% by mass or more and 99.99% by mass or less in a total amount of the gel.

3. The gel according to claim 1, further comprising: one or more components selected from the group consisting of gel-forming polymers, saccharides, sugar alcohols, alcohols, polyhydric alcohols, water-soluble polymers, and basic substances.

4. The gel according to claim 1, wherein the bagworm silk is silk spun by Eumeta japonica, Eumeta minuscula, or Nipponopsyche fuscescens.

5. A food, a pharmaceutical product, a cosmetic product, a sanitary material, a biomaterial, a cushioning material, an elastic material, a fishing tool, a medical device, or an industrial material comprising: the gel according to any one of claims 1 to 4.

6. A method for producing the gel according to claim 1, comprising: heating a bagworm silk-containing aqueous solution to 50°C or higher, and subsequently cooling it.
